# EUROPÄISCHE PATENTSCHRIFT

(11) **EP 2 934 278 B1**
(45) Veröffentlichungstag und Bekanntmachung des Hinweises auf die Patenterteilung: **10.10.2018**
(21) Anmeldenummer: 13828770.1
(22) Anmeldetag: 12.12.2013
(51) Int. Cl.: A61B 1/04, A61B 1/06, A61B 90/30, A61B 34/30, A61B 90/00, A61B 1/00, A61B 1/05, A61B 1/313, A61B 1/32, A61B 17/34

(54) **ENTKOPPELTES MEHRKAMERASYSTEM FÜR DIE MINIMAL-INVASIVE CHIRURGIE**
DECOUPLED MULTI-CAMERA SYSTEM FOR MINIMALLY INVASIVE SURGERY
SYSTÈME MULTICAMÉRA DÉCOUPLÉ POUR LA CHIRURGIE MINIMALEMENT INVASIVE

(30) Priorität: 20.12.2012 DE 102012025100
(43) Veröffentlichungstag der Anmeldung: 28.10.2015
(73) Patentinhaber: avateramedical GmbH, 07745 Jena (DE)
(72) Erfinder: VON GRÜNBERG, Hubertus, 30625 Hannover (DE); SEEBER, Marcel, 07745 Jena (DE)
(74) Vertreter: Ascherl, Andreas
(86) Internationale Anmeldenummer: PCT/DE2013/000804
(87) Internationale Veröffentlichungsnummer: WO 2014/094717

(56) Entgegenhaltungen:
- WO-A2-2009/057117
- US-A1- 2009 137 868
- US-A1- 2009 248 041

## Beschreibung

Die vorliegende Erfindung betrifft ein Mehrkamerasystem bestehend aus mindestens einer Endoskop-Kamera und mindestens einer Trokar-Kamera für den Einsatz bei minimal-invasiven Eingriffen sowie einem entsprechenden chirurgischen Roboter, vor allem für den Einsatz in der minimal-invasiven Chirurgie, wie der Laparoskopie.

Minimal-invasive chirurgische Eingriffe, wie laparoskopische Operationen, erfolgen über den Einsatz von chirurgischen Instrumenten, wie beispielsweise Greifzangen, Schneidwerkzeugen und Nähwerkzeugen, die über einen bzw. mehrere Trokare in das Körperinnere eines Patienten eingeführt werden. In der Regel kommen zwei bis vier, in den meisten Fällen drei, chirurgische Instrumente zum Einsatz. Neben diesen chirurgischen Instrumenten ist es erforderlich, dass eine Visualisierungseinheit vorhanden ist, die dem Chirurgen eine Beobachtung des Operationsgebiets ermöglicht. Eine solche Visualisierungseinheit ist regelmäßig eine Kamera bzw. ein Endoskop, die/das ebenfalls über einen Trokar in das Körperinnere des Patienten eingeführt wird. Üblicherweise erfolgt die Visualisierung über ein Endoskop, welches Aufnahmen des Operationsgebiets in 2D oder 3D auf einem externen Monitor darstellt. Im Stand der Technik sind zahlreiche Endoskope bekannt, bei welchen eine Visualisierungseinheit, wie einer Kamera, in ihr distales Ende integriert sind. Allgemein können Endoskope eine Kamera jedoch sowohl an ihrem distalen als auch an ihrem proximalen Ende aufweisen. Die mit dem Endoskop erzielten Aufnahmen werden über ein Bildweiterleitungssystem und einer Bildverarbeitungseinheit auf einem oder mehreren externen Monitoren abgebildet. Zahlreiche Endoskope werden im Stand der Technik beschrieben.

So beschreibt beispielsweise die WO2009/057117A2 ein Endoskop mit zwei bildgebenden Vorrichtungen. Die bildgebenden Vorrichtungen werden über einen Trokar in das Körperinnere geführt und über Klappen, die an dem Trokar befestigt sind, seitlich ausgeklappt und zwar winkelförmig von der Längsachse des Trokars zur Seite. Beide bildgebenden Vorrichtungen können mit einem unterschiedlichen Winkel ausgeschwenkt werden, so dass zwei verschiedene Aufnahmen erzielt werden können.

US 2009/0137868 beschreibt ebenfalls ein Endoskopsystem mit einem ersten und einem zweiten Endoskop als bildgebende Vorrichtungen. Hierbei nimmt die zweite bildgebende Vorrichtung eine Aufnahme eines Subjekts aus einer anderen Richtung auf, als die erste bildgebende Vorrichtung. Das Subjekt kann dadurch aus zwei unterschiedlichen Richtungen beobachtet werden.

US 2009/0248041 beschreibt ein chirurgisches Robotersystem, welches eine bildgebende Vorrichtung, kombiniert mit einem Laser zum Schneiden von Gewebe umfasst. Dieses Werkzeug ist an einem Roboterarm befestigt und mit einer Kontrolleinheit gekoppelt, an welche es Aufnahmen des zu operierenden Gewebes übermittelt.

Die Nachteile der im Stand der Technik beschriebenen Kamerasysteme bzw. Endoskope ist, dass das Endoskop nur für die Visualisierung des Operationsgebiets vorgesehen ist, dieses Endoskop jedoch nicht die Lage und Orientierung der in den Bauchraum eingeführten chirurgischen Instrumente gleichzeitig abbilden kann, bedingt durch die variierenden Positionen der chirurgischen Instrumente und der Position des Endoskops nahe am Operationsgeschehen sowie des Objektfeldwinkels (FoV, "Field of View"), wobei lediglich der unmittelbare Bereich des operativen Geschehens abgebildet wird. Wird ein chirurgisches Instrument aus dem Operationssichtfeld entfernt, wird es vom Endoskop nicht mehr erfasst und steht nicht mehr unter der visuellen Kontrolle des Chirurgen oder dessen Assistenten.

Der Erfindung liegt demnach die Aufgabe zugrunde, ein verbessertes Visualisierungssystem für minimal-invasive chirurgische Eingriffe, wie laparoskopische Eingriffe, zur Verfügung zu stellen, welches es dem Operateur die Zusatzinformation über die Lage und Orientierung der in den Patienten über z.B. den Bauchraum eingeführten Instrumente liefert.
Diese Aufgabe wird durch die vorliegende Erfindung gemäß Anspruch 1 durch ein chirurgisches Robotersystem mit zumindest zwei entsprechenden Endoskopen gelöst.

Die vorliegende Erfindung stellt ein entkoppeltes Mehrkamerasystem, bestehend aus einem Endoskop und mindestens einer weiteren Trokar-Kamera für den Einsatz bei minimal-invasiven chirurgischen Eingriffen, wie der Laparoskopie, dar.

Ein erster Gegenstand der vorliegenden Erfindung betrifft eine Trokar-Kamera für die minimalinvasive Chirurgie, insbesondere zur Verwendung innerhalb eines chirurgischen Robotersystems mit zumindest zwei Roboterarmen, an welchen jeweils zumindest ein Endoskop für die minimalinvasive Chirurgie angeordnet sind,
wobei das erste Endoskop an dem ersten Roboterarm eine erste Hauptträgereinrichtung, die sich im Wesentlichen über die gesamte Endoskoplänge von außen in das Körperinnere erstreckt, und welche am distalen Ende zumindest eine Beleuchtungseinheit und zwei Bildaufnahmeeinrichtungen aufweist, wobei die Bildaufnahmeeinrichtungen jeweils im Wesentlichen in der gleichen Ebene von der ersten Hauptträgereinrichtung nach außen schwenkbar angeordnet sind, und einen ersten Trokar umfasst, welcher den Zugang des ersten Endoskops in das Körperinnere bewerkstelligt, und
wobei das zweite Endoskop an dem zweiten Roboterarm eine zweite Hauptträgereinrichtung für ein chirurgisches Instrument, die sich im Wesentlichen über die gesamte Endoskoplänge von außen in das Körperinnere erstreckt,
einen zweiten Trokar, welcher den Zugang des zweiten Endoskops in das Körperinnere bewerkstelligt, und
eine erste Zusatzträgereinrichtung aufweist, die der zweiten Hauptträgereinrichtung vorgesehen ist, wobei die Zusatzträgereinrichtung an ihrem distalen Ende eine Zusatzbildaufnahmeeinrichtung aufweist, die von der Zusatzträgereinrichtung nach außen schwenkbar angeordnet ist und wobei die Zusatzbildaufnahmeeinrichtung eine Zusatzbeleuchtungseinheit und zumindest einen Zusatzbildsensor aufweist, welcher einen Überwachungsbereich aufweist, der die beiden Überwachungsbereiche der Bildaufnahmeeinrichtungen des ersten Endoskops umfasst,
wobei eine Bildverarbeitungseinheit, welche sowohl mit den beiden Bildaufnahmeeinrichtungen als auch der Zusatzbildaufnahmeeinrichtung gekoppelt ist, und eine Visualisierungseinheit vorgesehen ist, welche 2D-Bilddaten und/oder 3D-Bilddaten der Bildaufnahmeeinrichtungen und/oder der Zusatzbildaufnahmeeinrichtung darstellt
wobei die Zusatzträgereinrichtung (3) zwischen dem Trokar (1a) und der zweiten Hauptträgereinrichtung (4a) vorgesehen ist, insbesondere unmittelbar an der zweiten Hauptträgereinrichtung (4a) anliegt, wobei insbesondere sowohl die zweite Hauptträgereinrichtung (4a) als auch die Zusatzträgereinrichtung (3) zylinderförmig ausgebildet sind.

Die vorliegende Erfindung hat den Vorteil, dass durch die Bereitstellung und gleichzeitiger Nutzung von mindestens 2 bildgebenden Systemen, mindestens einer zumindest 2D-Übersichtskamera an einem Endoskop und einer 3D-Detailkamera an einem weiteren Endoskop, wobei für die Zuführung der 2D-Übersichtskamera ein Kombinations-Trokar gemeinsam mit einem chirurgischen Instrument genutzt wird und in das Körperinnere eines Patienten eingeführt werden, es möglich ist, sowohl ein zumindest ein 2D-Übersichtsbild mit einem hohen Objektfeldwinkel (Weitwinkel von typischerweise > 90°) als auch ein 3D-Detailbild mit einem üblichen Objektfeldwinkel von bis zu 70° zu generieren. Dies ermöglicht es, während der gesamten Dauer eines minimal-invasiven chirurgischen, wie laparoskopischen, Eingriffs, das direkte Operationsgebiet sowie dessen weiteren Umkreis abzubilden. Auf diese Weise können alle chirurgischen Instrumente gleichzeitig abgebildet werden, auch wenn Sie sich, bedingt durch ihre variierenden Positionen und der Position des Endoskops sowie des Objektfeldwinkels (FoV, "Field of View") außerhalb des Operationssichtfeldes des Endoskop befinden, da die Zusatzbildaufnahmeeinrichtung auch Instrumente erfassen kann, welche sich außerhalb des Operation Sichtfeldes des Endoskopes befinden. Dies kann beispielsweise der Fall sein, wenn ein chirurgisches Instrument zeitweise nicht benötigt "geparkt" wird. Dieses "Parken" erfolgt in den meisten Fällen außerhalb des direkten Operationsgeschehens und außerhalb des Operationssichtfeld, damit es bei dem Eingriff nicht im Wege ist. Erfindungsgemäß werden solche "geparkten" chirurgischen Elemente von der erfindungsgemäßen 2D-Übersichtskamera erfasst und stehen so kontinuierlich unter der visuellen Kontrolle des Chirurgen oder dessen Assistenten.

Ferner wird durch die Zusatzbeleuchtungseinheit an der Zusatzträgereinrichtung insbesondere für die 3D-Aufnahmen eine verbesserte Ausleuchtung erzielt, so dass die Bilder der 3D-Detailkamera qualitativ verbessert dargestellt werden können.

Gemäß einer bevorzugten Ausführungsform der Erfindung weist der Zusatzbildsensor eine Weitwinkeloptik auf, welcher im ausgeschwenkten Zustand nahe an dem distalen Ende des zweiten Trokars angeordnet ist.

Weiterin offenbart ist ein chirurgisches Robotersystem mit zumindest zwei Roboterarmen, an welchem ein chirurgisches Instrument und ein Endoskop für die minimalinvasive Chirurgie anordenbar ist, wobei an dem Trokar für das chirurgische Instrument zusätzlich die Zusatzträgereinrichtung mit der Zusatzbildaufnahmeeinrichtung angeordnet ist.,
einen Trokar, welcher den Zugang des Endoskops in das Körperinnere bewerkstelligt, und und die wobei die Zusatzträgereinrichtung an ihrem distalen Ende eine Zusatzbildaufnahmeeinrichtung aufweist, die von der Zusatzträgereinrichtung nach außen schwenkbar angeordnet ist und wobei die Zusatzbildaufnahmeeinrichtung eine Zusatzbeleuchtungseinheit und zumindest einen Zusatzbildsensor aufweist, welcher einen Überwachungsbereich aufweist, der alle z.B. über den Bauchraum eingeführten chirurgischen Instrumente und/oder Endoskope in ihrer Lage und Orientierung umfasst,
wobei eine Bildverarbeitungseinheit, welche sowohl mit dem Endoskop als auch der Zusatzbildaufnahmeeinrichtung gekoppelt ist, und eine Visualisierungseinheit vorgesehen ist, welche 2D-Bilddaten und/oder 3D-Bilddaten des Endoskopes und/oder der Zusatzbildaufnahmeeinrichtung darstellt.
wobei einer Steuereinheit die aktuelle Position und Orientierung der Roboterarme und der daran befestigten Instrumente oder Endoskope bekannt ist und diese Information an eine Bildverarbeitungseinheit, welche sowohl mit dem Endoskop als auch der Zusatzbildaufnahmeeinrichtung gekoppelt ist, und eine Visualisierungseinheit vorgesehen ist, welche 2D-Bilddaten und/oder 3D-Bilddaten des Endoskopes und/oder der Zusatzbildaufnahmeeinrichtung darstellt und zusätzlich aus der Position und Orientierung der Roboterarme und der daran befestigten Instrumente oder Endoskope deren Bewegungs-Trajektorien berechnen und als Overlay-Darstellung zusammen mit den 2D-Bilddaten und/oder 3D-Bilddaten darstellt.
Das erfindungsgemäße chirurgische Robotersystem weist insbesondere den Vorteil auf, dass die Bilddaten für den Chirurgen je nach Bedarf als 2D-Bilddaten und/oder 3D-Bilddaten dargestellt werden können, d.h., dass die Bilddaten der Übersichtskamera auch mit den Bilddaten der 3D-Detailkamera mithilfe der Bildbearbeitungseinheit gekoppelt werden können, um so dem Chirurgen eine stark verbesserte Übersicht durch eine einzige Bildabfolge auf der Visualisierungseinheit zu ermöglichen.
So ist es insbesondere von Vorteil, wenn der Zusatzbildsensor eine Weitwinkeloptik aufweist, welcher im ausgeschwenkten Zustand nahe an dem distalen Ende des Trokars angeordnet ist.
Insbesondere ist es von Vorteil, wenn die beiden Bildaufnahmeeinrichtungen jeweils am distalen Ende der ersten Hauptträgereinrichtung um eine Drehachse (Schwenkachse) schwenkbar angeordnet sind, wobei die Schwenkachsen parallel zueinander in einer Ebene liegen, wodurch der konstruktive Aufwand minimiert ist.

Eine weitere konstruktive Vereinfachung ist darin zu sehen, dass die Zusatzträgereinrichtung zwischen dem Trokar und der Hauptträgereinrichtung, insbesondere unmittelbar an der Hauptträgereinrichtung anliegt, wobei insbesondere sowohl die Hauptträgereinrichtung als auch die Zusatzträgereinrichtung zylinderförmig ausgebildet sind.

Ferner ist es von Vorteil, wenn die Bildaufnahmeeinrichtungen mittels Gelenken jeweils sowohl um die Schwenkachse als auch um eine weitere Drehachse orthogonal zur Längserstreckung der Trägereinrichtung kippbar angeordnet sind, wobei die Drehbewegungen um die Schwenkachsen und die Drehachsen voneinander unabhängig entkoppelt sind.

Eine weitere bevorzugte Ausführungsform der Erfindung ist dadurch gekennzeichnet, dass zumindest ein drittes Endoskop an einem dritten Roboterarm vorgesehen ist, welches eine dritte Hauptträgereinrichtung für ein chirurgisches Instrument, die sich im Wesentlichen über die gesamte Endoskoplänge von außen in das Körperinnere erstreckt, einen dritten Trokar, welcher den Zugang des dritten Endoskops in das Körperinnere bewerkstelligt, und eine zweite Zusatzträgereinrichtung aufweist, die an der dritten Hauptträgereinrichtung vorgesehen ist, wobei die zweite Zusatzträgereinrichtung an ihrem distalen Ende eine zweite Zusatzbildaufnahmeeinrichtung aufweist, die von der Zusatzträgereinrichtung nach außen schwenkbar angeordnet ist und wobei die zweite Zusatzbildaufnahmeeinrichtung eine Zusatzbeleuchtungseinheit und zumindest einen zweiten Zusatzbildsensor aufweist, welcher einen zweiten Überwachungsbereich aufweist, der die beiden Überwachungsbereiche der beiden Bildaufnahmeeinrichtungen des ersten Endoskops umfasst.

Ferner ist es vorteilhaft, wenn die zweite Zusatzbildaufnahmeeinrichtung des dritten Endoskops mit der Bildverarbeitungseinheit gekoppelt ist, und die Visualisierungseinheit die 2D-Bilddaten und/oder 3D-Bilddaten der beiden Bildaufnahmeeinrichtungen und/oder der ersten Zusatzbildaufnahmeeinrichtung und/oder der zweiten Zusatzbildaufnahmeeinrichtung darstellt.

Die gesamte Offenbarung der vorliegenden Erfindung bezieht sich demnach gleichermaßen sowohl auf die Kombination, bestehend aus einer 3D-Detailkamera und mindestens einer weiteren 2D-Übersichtskamera die vorzugsweise über einen weiteren, für ein chirurgisches Instrument genutzten Trokar, in das Körperinnere eingeführt wird und dabei so positioniert wird, dass alle über weitere Trokare in den Körper eingeführten chirurgischen Instrumente bzw. Endoskop-Kameras von der 2D-Übersichtskamera optisch erfasst werden,
als auch auf die Kombination einer 3D-Detailkamera mit mindestens zwei 2D-Übersichtskameras die vorzugsweise über zwei weitere, für chirurgische Instrumente genutzte Trokare, in das Körperinnere eingeführt werden und dabei so positioniert sind, dass alle über weitere Trokare in den Körper eingeführten chirurgischen Instrumente bzw. Endoskop-Kameras von den 2D-Übersichtskamera optisch erfasst werden.

Bei minimal-invasiven chirurgischen Eingriffen, wie laparoskopischen Eingriffen, wird über einen Trokar ein Zugang in das Körperinnere eines Patienten (üblicherweise durch die Bauchdecke oder in den Brustraum) geschaffen. Durch einen solchen Trokar kann ein chirurgisches Instrument oder eine Kamera bzw. ein Endoskop in das Körperinnere geführt werden. Wie erwähnt, wird erfindungsgemäß über einen Trokar ein chirurgisches Instrument und eine Trokar-Kamera gleichzeitig eingeführt. Da in der Regel für einen Eingriff 2 bis 4 chirurgische Instrumente und mindestens eine Kamera benötigt werden, sind 3 bis 5 Trokare erforderlich.

Rein beispielhaft wird die vorliegende Erfindung anhand der beigefügten Figuren erläutert. Es zeigt:

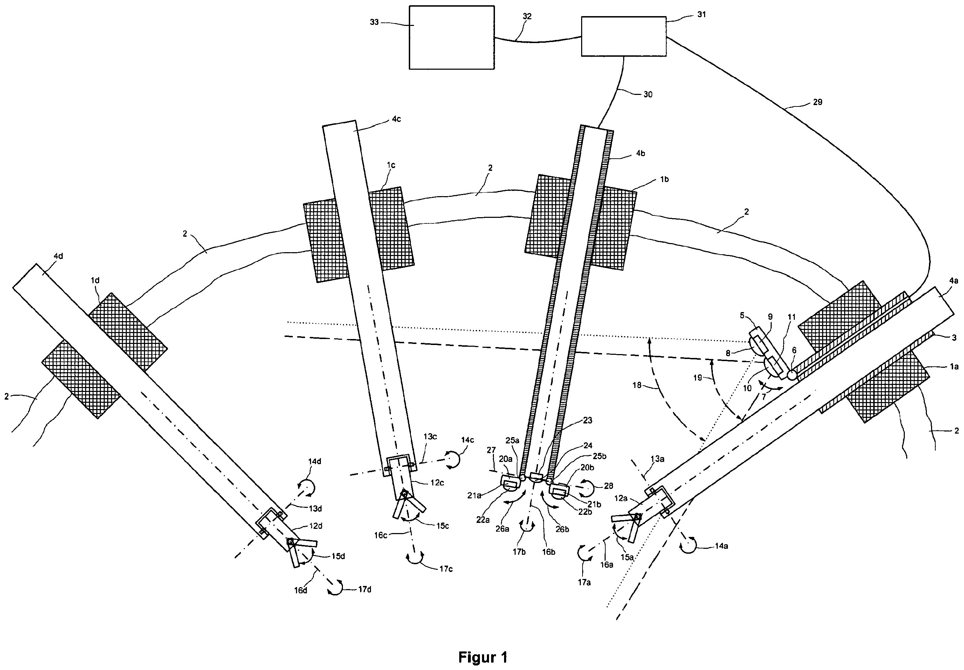

Figur 1 eine schematische Ansicht einer bevorzugten Trokaranordnung während eines minimal-invasiven Eingriffs unter Verwendung eines bestimmungsgemäßen Endoskops in einer bevorzugten Ausführungsform einer 3D-Detailkamera, welche an einem erfindungsgemäßen Endoskop angeordnet ist, und einer mindestens 2D-Übersichtskamera, welche an einem separaten Träger an einem weiteren Trokar angeordnet ist, welche mit einer Bildverarbeitungseinheit und einer Visualisierungseinheit eines chirurgischen Robotersystems verbunden sind, und

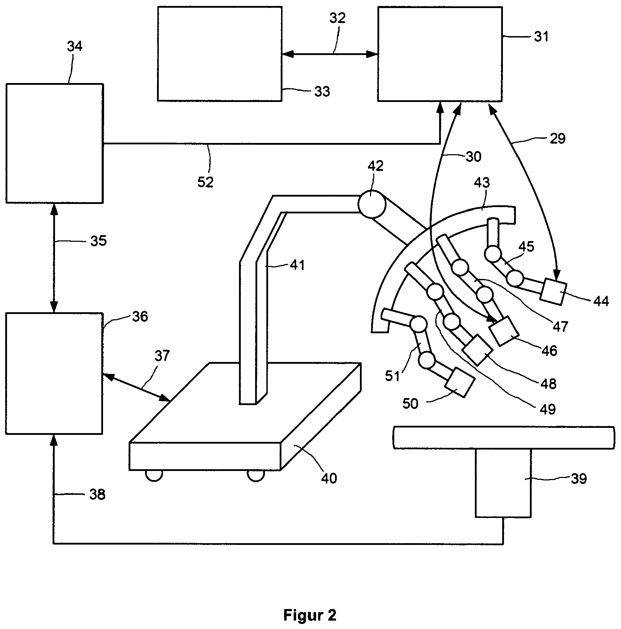

Figur 2 eine schematische Gesamtansicht der Verwendung der Visualisierungslösung, bestehend aus 3D-Detailkamera und 2D-Übersichtskamera, in einem chirurgischen Robotersystem für den Einsatz in der minimal-invasiven Chirurgie, wie z.B. der Laparoskopie, und

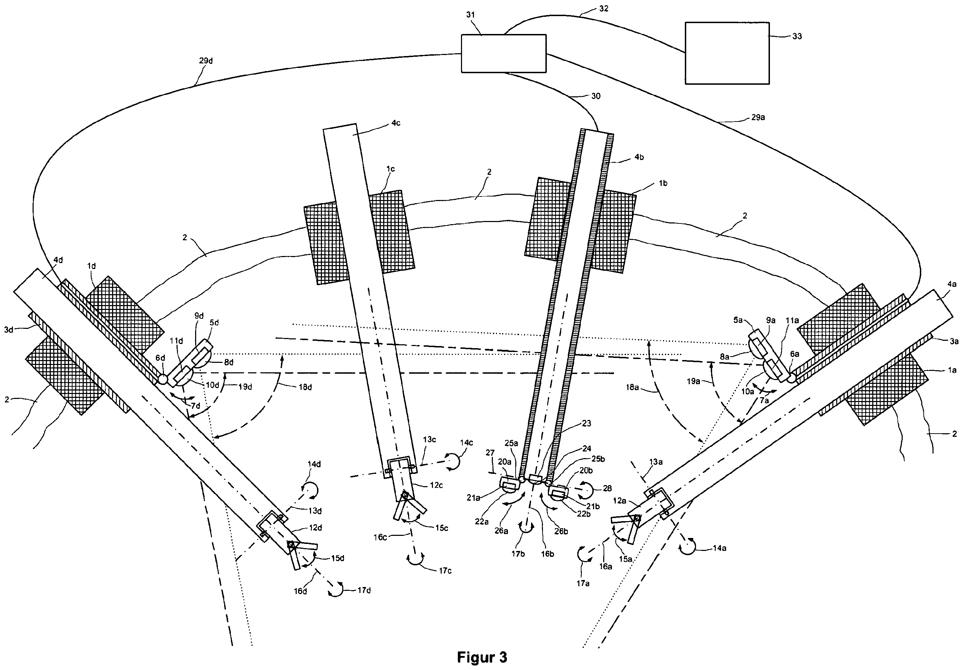

Figur 3 eine schematische Ansicht einer bevorzugten Trokaranordnung während eines minimal-invasiven Eingriffs unter Verwendung eines bestimmungsgemäßen Endoskops in einer bevorzugten Ausführungsform einer 3D-Detailkamera, welche an einem erfindungsgemäßen Endoskop angeordnet ist, und von mindestens zwei 2D-Übersichtskameras welches, welche an separaten Trägern an zwei verschiedenen weiteren Trokaren angeordnet sind, welche mit einer Bildverarbeitungseinheit und einer Visualisierungseinheit eines chirurgischen Robotersystems verbunden sind, und

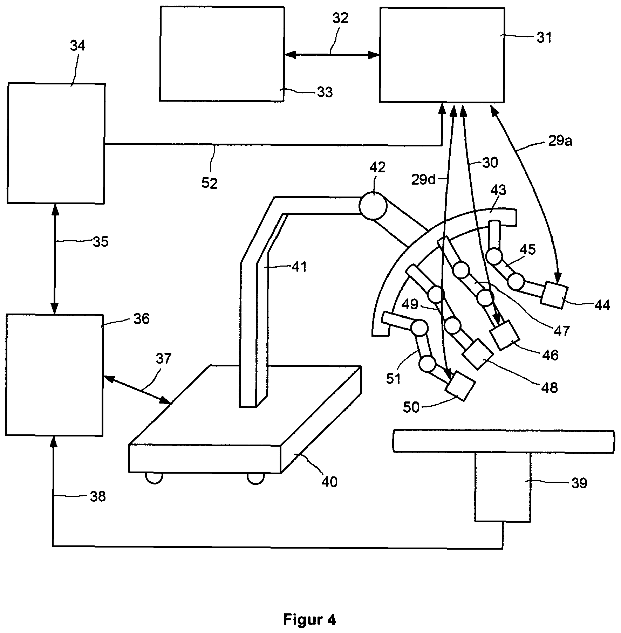

Figur 4 eine schematische Gesamtansicht der Verwendung der Visualisierungslösung, bestehend aus 3D-Detailkamera und zwei 2D-Übersichtskameras, in einem chirurgischen Robotersystem für den Einsatz in der minimal-invasiven Chirurgie, wie z.B. der Laparoskopie.

**Figur 1** zeigt das erfindungsgemäße Mehrkamerasystem. Über einen Trokar 1a wird eine Durchführung durch das Körpergewebe 2 und somit ein Zugang in das Körperinnere eines Patienten geschaffen. Durch den Trokar 1a wird ein Zusatzträger 3 für eine 2D-Übersichtskamera in den Körper eingeführt. Der Zusatzträger 3 ist so gestaltet, dass dieser eine röhrenförmige Durchführung für einen rotationssymmetrischen, stabförmig gestalteten weiteren Hauptträger 4a für ein chirurgisches Instrument ermöglicht. An dem Zusatzträger 3 ist über ein Gelenk 6 ein Kamerahalter 5 so befestigt, dass dieser in wesentlichen um 90° zur Rotationsachse durch eine Schwenkbewegung 7 nach Durchführung durch den Trokar 1a ausgeklappt werden kann. Der Kamerahalter 5 trägt einen Zusatzbildsensor, bestehend aus Bildsensor 9 und Weitwinkel-Abbildungsoptik 8 mit dem Öffnungswinkel 18. Um das Objektfeld auszuleuchten ist der Kamerahalter 5 weiterhin mit einer Zusatzbeleuchtungseinheit, bestehend aus einer Lichtquelle 11 und einer entsprechenden Weitwinkel-Abbildungsoptik 10 mit dem Öffnungswinkel 19, ausgestattet. Diese Weitwinkel-Abbildungsoptik 10 ist derart gestaltet, das bis auf den Parallaxen-Versatz zwischen der Weitwinkel-Abbildungsoptik 8 und Weitwinkel-Abbildungsoptik 10, das komplette vom Bildsensor 9 und der damit verbundenen Weitwinkel-Abbildungsoptik 8 erfasste Objektfeld, ausgeleuchtet wird. Der Kamerahalter 5 mit dem Zusatzbildsensor und der Zusatzbeleuchtungseinheit bilden zusammen die 2D-Übersichtskamera zur Erzeugung eines 2D-Übersichtsbildes. Vorzugsweise ist der Bildsensor 9 ausgeprägt als CCD- oder CMOS-Sensor mit einer Auflösung von 1920x1080 Bildpunkten oder höher. Durch eine geeignete Positionierung der 2D-Übersichtskamera an einem äußeren Trokar 1a befinden sich alle weiteren in den Körper über die Trokare 1b, 1c, 1d eingeführten chirurgischen Instrumente bzw. Endoskope im Objektfeld der 2D-Übersichtskamera und können durch diese optisch erfasst und auf dem Bildsensor 9 abgebildet werden.

Die aufgenommen Bilddaten werden über die Datenstrecke 29 einer Verarbeitungseinheit 31 zugeführt, welche die Bilddaten zur Darstellung aufbereitet und über eine weitere Datenstrecke 32 einer Visualisierungseinheit 33 zuführt. Die Visualisierungseinheit 33 kann sowohl 2D- als auch 3D-Bilddaten darstellen, beispielsweise getrennt, jedoch auch kombiniert in einem einzigen Bild bzw. einer einzigen Bildabfolge.

Am Ende des rotationssymmetrischen Hauptträgers 4b befinden sich 2 Kameramodule bzw. zwei Bildaufnahmeeinrichtungen 20a, 21a, 22a, 20b, 21b, 22b bestehend insbesondere aus jeweils 2 Abbildungsoptiken 22a und 22b montiert auf 2 Kamerahaltern 20a und 20b. Die Kamerahalter 20a und 20b sind über die Gelenke 25a und 25b, welche die Schwenkachsen bilden, derart mit dem Hauptträger 4b verbunden, dass diese um 90° zur Rotationsachse des Hauptträgers 4b nach Einführung in den Körper in Schwenkrichtung 26a bzw. 26b ausgeklappt werden können. Um das Objektfeld zu beleuchten, ist an dem Ende des Hauptträgers 4b an dem auch die ausklappbaren Kamerahalter 20a und 20b befestigt sind, eine Beleuchtungseinheit, bestehend aus der Lichtquelle 23 und einer Abbildungsoptik 24, montiert. Die Kamerahalter 20a und 20b tragen weiterhin Bildaufnehmer, bestehend aus Bildsensoren 21a und 21b und Abbildungsoptiken 22a und 22b. Zusammen bilden diese beiden Bildaufnahmeeinrichtungen 20a, 21a, 22a, 20b, 21b, 22b die 3D-Detailkamera.
Die Beleuchtungseinheit, bestehend aus Lichtquelle 23 und Abbildungsoptik 24, kann vorzugsweise ausgeführt werden als direkte LED-Lichtquelle, derart gestaltet, das der Abstrahlwinkel der LED, in Verbindung mit einer geeigneten Abbildungsoptik 24, so gewählt wird, das das von beiden Bildsensoren 21a und 21b und den damit verbundenen Abbildungsoptiken 22a und 22b abgebildete Objektfeld, vollständig ausgeleuchtet wird.

Die aufgenommen Bilddaten werden über die Datenstrecke 30 einer Verarbeitungseinheit 31 zugeführt, welche die Bilddaten zur Darstellung aufbereitet und über eine weitere Datenstrecke 32 einer Visualisierungseinheit 33 zuführt. Die Visualisierungseinheit 33 kann sowohl 2D- als auch 3D-Bilddaten darstellen, beispielsweise getrennt, jedoch auch kombiniert in einem einzigen Bild bzw. einer einzigen Bildabfolge.

**Figur 2** zeigt die Verwendung der Visualisierungslösung, bestehend aus 3D-Detailkamera und 2D-Übersichtskamera, in einem chirurgischen Robotersystem für den Einsatz in der minimal-invasiven Chirurgie, wie z.B. der Laparoskopie. Dargestellt ist eine Ausführungsform des erfindungsgemäßen Robotersystems mit 4 Roboterarmen 45, 47, 49, 51 und 4 Trokar-Zugängen 44, 46, 48, 50, wobei 44 die in Figur 1 dargestellte 2D-Übersichtskamera am Trokar 1a, 46 die in Figur 1 dargestellte 3D-Detailkamera am Trokar 1b, 48 und 50 die Trokare 1c, 1d für den Zugang von zwei weiteren chirurgischen Instrumenten 4c, 4d, beinhaltet. Der Zugang 44 für die 2D-Übersichtskamera wird mittels einer Vorpositionierungseinrichtung 45 mit der Bogenführung 43 verbunden. Der Zugang 46 für die 3D-Detailkamera wird mittels einer Vorpositionierungseinrichtung 47 mit der Bogenführung 43 verbunden. Der Zugang 48 für ein chirurgisches Instrument wird mittels einer Vorpositionierungseinrichtung 49 mit der Bogenführung 43 verbunden. Der Zugang 50 für ein chirurgisches Instrument wird mittels einer Vorpositionierungseinrichtung 51 mit der Bogenführung 43 verbunden. Die Vorpositionierungseinrichtung kann passiv, d.h. durch manuelle Verstellung oder auch aktiv realisiert sein. Die Vorpositionierungseinrichtung selbst wird mittels einer geeigneten Halterung, z.B. als Bogenführung 43 gehaltert. Diese Bogenführung 43 kann mittels des Gelenkes 42 zum Patienten positioniert werden. Der Ausleger 41 ist verbunden mit dem fahrbaren Trägersystem 40 und ermöglicht damit eine Positionierung des gesamten Trägersystems relativ zum OP-Tisch 39. Über die Bedien- und Anzeigeeinheit 34 wird dem Bediener der aktuelle Status der Vorpositioniereinrichtung ausgegeben. Über die Bedien- und Anzeigeeinheit 34 kann der Bediener Steuerbefehle eingeben, welche über eine geeignete Datenverbindung 35 an die Steuereinheit 36 und von dieser an den Zugang für die 2D-Übersichtskamera 44, an den Zugang für die 3D-Detailkamera 46, an die Zugänge 48, 50, an die Vorpositioniereinrichtung 45, 47, 49, 51 sowie an die Bogenführung 43 zur weiteren Verarbeitung gesendet werden. Die Steuereinheit 36 ist über eine geeignete Datenverbindung 37 mit dem Trägersystem verbunden. Der OP-Tisch 39 kann steuerungstechnisch über die Datenverbindung 38 ebenfalls mit der Steuereinheit 36 verbunden sein, um bei einer Veränderung der OP-Tisch - Position, z.B. der Höhe, diese Positionsänderung in der Steuereinheit zu verarbeiten und zu signalisieren. Damit können Veränderungen der Patientenposition aufgrund einer Positionsveränderung des OP-Tisches 39 ausgewertet werden.

Die aufgenommen Bilddaten werden über die Datenstrecken 29, 30 einer Verarbeitungseinheit 31 zugeführt, welche die Bilddaten zur Darstellung aufbereitet und über eine weitere Datenstrecke 32 einer Visualisierungseinheit 33 zuführt. Die Visualisierungseinheit 33 kann sowohl 2D- als auch 3D-Bilddaten darstellen, beispielsweise getrennt, jedoch auch kombiniert in einem einzigen Bild bzw. einer einzigen Bildabfolge.

Die Bedien- und Anzeigeeinheit 34 ist über eine geeignete Datenverbindung 52 mit der Verarbeitungseinheit 31 gekoppelt. Der Chirurg kann über die Bedien- und Anzeigeeinheit 34 Steuerbefehle zur Auswahl, Verarbeitung und Darstellung der Bilddaten an die Verarbeitungseinheit 31 senden. Die Verarbeitungseinheit 31 ist mittels einer geeigneten Datenverbindung 32 mit der Anzeigeeinheit 33 verbunden. Die Anzeigeeinheit 33 kann die von der 2D-Übersichtskamera und der 3D-Detailkamera gelieferten Bilder / Bildfolgen sowie zusätzliche, in der Verarbeitungseinheit 31 erzeugte Informationen, wie z.B. Trajektorien der chirurgischen Instrumente, entweder als separate Bilder / Bildfolge oder als mit den Bildinformationen der 2D-Übersichtskamera und/oder der 3D-Detailkamera verrechnete Bilder / Bildfolgen darstellen.

**Figur 3** zeigt das erfindungsgemäße Mehrkamerasystem. Über einen Trokar 1a wird eine Durchführung durch das Körpergewebe 2 und somit ein Zugang in das Körperinnere eines Patienten geschaffen. Durch den Trokar 1a wird ein Zusatzträger 3a für eine erste 2D-Übersichtskamera in den Körper eingeführt. Der Zusatzträger 3a ist so gestaltet, dass dieser eine röhrenförmige Durchführung für einen rotationssymmetrischen, stabförmig gestalteten weiteren Hauptträger 4a für ein chirurgisches Instrument ermöglicht. An dem Zusatzträger 3a ist über ein Gelenk 6a ein Kamerahalter 5a so befestigt, dass dieser in wesentlichen um 90° zur Rotationsachse durch eine Schwenkbewegung 7a nach Durchführung durch den Trokar 1a ausgeklappt werden kann. Der Kamerahalter 5a trägt einen Zusatzbildsensor, bestehend aus Bildsensor 9a und Weitwinkel-Abbildungsoptik 8a mit dem Öffnungswinkel 18a. Um das Objektfeld auszuleuchten ist der Kamerahalter 5a weiterhin mit einer Zusatzbeleuchtungseinheit, bestehend aus einer Lichtquelle 11a und einer entsprechenden Weitwinkel-Abbildungsoptik 10a mit dem Öffnungswinkel 19a, ausgestattet. Diese Weitwinkel-Abbildungsoptik 10a ist derart gestaltet, das bis auf den Parallaxen-Versatz zwischen der Weitwinkel-Abbildungsoptik 8a und Weitwinkel-Abbildungsoptik 10a, das komplette vom Bildsensor 9a und der damit verbundenen Weitwinkel-Abbildungsoptik 8a erfasste Objektfeld, ausgeleuchtet wird. Der Kamerahalter 5a mit dem Zusatzbildsensor und der Zusatzbeleuchtungseinheit bilden zusammen die erste 2D-Übersichtskamera zur Erzeugung eines ersten 2D-Übersichtsbildes. Vorzugsweise ist der Bildsensor 9a ausgeprägt als CCD- oder CMOS-Sensor mit einer Auflösung von 1920x1080 Bildpunkten oder höher. Durch eine geeignete Positionierung der ersten 2D-Übersichtskamera an einem äußeren Trokar 1a befinden sich alle weiteren in den Körper über die Trokare 1b, 1c, 1d eingeführten chirurgischen Instrumente bzw. Endoskope im Objektfeld der ersten 2D-Übersichtskamera und können durch diese optisch erfasst und auf dem Bildsensor 9a abgebildet werden.

Die aufgenommen Bilddaten werden über die Datenstrecke 29a einer Verarbeitungseinheit 31 zugeführt, welche die Bilddaten zur Darstellung aufbereitet und über eine weitere Datenstrecke 32 einer Visualisierungseinheit 33 zuführt. Die Visualisierungseinheit 33 kann sowohl 2D- als auch 3D-Bilddaten darstellen, beispielsweise getrennt, jedoch auch kombiniert in einem einzigen Bild bzw. einer einzigen Bildabfolge.

Über einen Trokar 1d wird eine Durchführung durch das Körpergewebe 2 und somit ein Zugang in das Körperinnere eines Patienten geschaffen. Durch den Trokar 1d wird ein Zusatzträger 3d für eine zweite 2D-Übersichtskamera in den Körper eingeführt. Der Zusatzträger 3d ist so gestaltet, dass dieser eine röhrenförmige Durchführung für einen rotationssymmetrischen, stabförmig gestalteten weiteren Hauptträger 4d für ein chirurgisches Instrument ermöglicht. An dem Zusatzträger 3d ist über ein Gelenk 6d ein Kamerahalter 5d so befestigt, dass dieser in wesentlichen um 90° zur Rotationsachse durch eine Schwenkbewegung 7d nach Durchführung durch den Trokar 1d ausgeklappt werden kann. Der Kamerahalter 5d trägt einen Zusatzbildsensor, bestehend aus Bildsensor 9d und Weitwinkel-Abbildungsoptik 8d mit dem Öffnungswinkel 18d. Um das Objektfeld auszuleuchten ist der Kamerahalter 5d weiterhin mit einer Zusatzbeleuchtungseinheit, bestehend aus einer Lichtquelle 11d und einer entsprechenden Weitwinkel-Abbildungsoptik 10d mit dem Öffnungswinkel 19d, ausgestattet. Diese Weitwinkel-Abbildungsoptik 10d ist derart gestaltet, das bis auf den Parallaxen-Versatz zwischen der Weitwinkel-Abbildungsoptik 8d und Weitwinkel-Abbildungsoptik 10d, das komplette vom Bildsensor 9d und der damit verbundenen Weitwinkel-Abbildungsoptik 8d erfasste Objektfeld, ausgeleuchtet wird. Der Kamerahalter 5d mit dem Zusatzbildsensor und der Zusatzbeleuchtungseinheit bilden zusammen die zweite 2D-Übersichtskamera zur Erzeugung eines zweiten 2D-Übersichtsbildes. Vorzugsweise ist der Bildsensor 9d ausgeprägt als CCD- oder CMOS-Sensor mit einer Auflösung von 1920x1080 Bildpunkten oder höher. Durch eine geeignete Positionierung der zweiten 2D-Übersichtskamera an einem äußeren Trokar 1d befinden sich alle weiteren in den Körper über die Trokare 1a, 1b, 1c eingeführten chirurgischen Instrumente bzw. Endoskope im Objektfeld der zweiten 2D-Übersichtskamera und können durch diese optisch erfasst und auf dem Bildsensor 9d abgebildet werden.

Die aufgenommen Bilddaten werden über die Datenstrecke 29d einer Verarbeitungseinheit 31 zugeführt, welche die Bilddaten zur Darstellung aufbereitet und über eine weitere Datenstrecke 32 einer Visualisierungseinheit 33 zuführt. Die Visualisierungseinheit 33 kann sowohl 2D- als auch 3D-Bilddaten darstellen, beispielsweise getrennt, jedoch auch kombiniert in einem einzigen Bild bzw. einer einzigen Bildabfolge.

Am Ende des rotationssymmetrischen Hauptträgers 4b befinden sich 2 Kameramodule bzw. zwei Bildaufnahmeeinrichtungen 20a, 21a, 22a, 20b, 21b, 22b bestehend insbesondere aus jeweils 2 Abbildungsoptiken 22a und 22b montiert auf 2 Kamerahaltern 20a und 20b. Die Kamerahalter 20a und 20b sind über die Gelenke 25a und 25b, welche die Schwenkachsen bilden, derart mit dem Hauptträger 4b verbunden, dass diese um 90° zur Rotationsachse des Hauptträgers 4b nach Einführung in den Körper in Schwenkrichtung 26a bzw. 26b ausgeklappt werden können. Um das Objektfeld zu beleuchten, ist an dem Ende des Hauptträgers 4b an dem auch die ausklappbaren Kamerahalter 20a und 20b befestigt sind, eine Beleuchtungseinheit, bestehend aus der Lichtquelle 23 und einer Abbildungsoptik 24, montiert. Die Kamerahalter 20a und 20b tragen weiterhin Bildaufnehmer, bestehend aus Bildsensoren 21a und 21b und Abbildungsoptiken 22a und 22b. Zusammen bilden diese beiden Bildaufnahmeeinrichtungen 20a, 21a, 22a, 20b, 21b, 22b die 3D-Detailkamera.
Die Beleuchtungseinheit, bestehend aus Lichtquelle 23 und Abbildungsoptik 24, kann vorzugsweise ausgeführt werden als direkte LED-Lichtquelle, derart gestaltet, das der Abstrahlwinkel der LED, in Verbindung mit einer geeigneten Abbildungsoptik 24, so gewählt wird, das das von beiden Bildsensoren 21a und 21b und den damit verbundenen Abbildungsoptiken 22a und 22b abgebildete Objektfeld, vollständig ausgeleuchtet wird.

Die aufgenommen Bilddaten werden über die Datenstrecke 30 einer Verarbeitungseinheit 31 zugeführt, welche die Bilddaten zur Darstellung aufbereitet und über eine weitere Datenstrecke 32 einer Visualisierungseinheit 33 zuführt. Die Visualisierungseinheit 33 kann sowohl 2D- als auch 3D-Bilddaten darstellen, beispielsweise getrennt, jedoch auch kombiniert in einem einzigen Bild bzw. einer einzigen Bildabfolge.

**Figur 4** zeigt die Verwendung der Visualisierungslösung, bestehend aus einer 3D-Detailkamera und zwei 2D-Übersichtskamera, in einem chirurgischen Robotersystem für den Einsatz in der minimal-invasiven Chirurgie, wie z.B. der Laparoskopie. Dargestellt ist eine Ausführungsform des erfindungsgemäßen Robotersystems mit 4 Roboterarmen 45, 47, 49, 51 und 4 Trokar-Zugängen 44, 46, 48, 50, wobei 44 die in Figur 3 dargestellte erste 2D-Übersichtskamera am Trokar 1a, 46 die in Figur 1 dargestellte 3D-Detailkamera am Trokar 1b, 50 die in Figur 3 dargestellte zweite 2D-Übersichtskamera am Trokar 1d und 48 den Trokare 1c für den Zugang eines weiteren chirurgischen Instrumentes 4c, beinhaltet. Der Zugang 44 für die erste 2D-Übersichtskamera wird mittels einer Vorpositionierungseinrichtung 45 mit der Bogenführung 43 verbunden. Der Zugang 46 für die 3D-Detailkamera wird mittels einer Vorpositionierungseinrichtung 47 mit der Bogenführung 43 verbunden. Der Zugang 48 für ein chirurgisches Instrument wird mittels einer Vorpositionierungseinrichtung 49 mit der Bogenführung 43 verbunden. Der Zugang 50 für die zweite 2D-Übersichtskamera wird mittels einer Vorpositionierungseinrichtung 51 mit der Bogenführung 43 verbunden. Die Vorpositionierungseinrichtung kann passiv, d.h. durch manuelle Verstellung oder auch aktiv realisiert sein. Die Vorpositionierungseinrichtung selbst wird mittels einer geeigneten Halterung, z.B. als Bogenführung 43 gehaltert. Diese Bogenführung 43 kann mittels des Gelenkes 42 zum Patienten positioniert werden. Der Ausleger 41 ist verbunden mit dem fahrbaren Trägersystem 40 und ermöglicht damit eine Positionierung des gesamten Trägersystems relativ zum OP-Tisch 39. Über die Bedien- und Anzeigeeinheit 34 wird dem Bediener der aktuelle Status der Vorpositioniereinrichtung ausgegeben. Über die Bedien- und Anzeigeeinheit 34 kann der Bediener Steuerbefehle eingeben, welche über eine geeignete Datenverbindung 35 an die Steuereinheit 36 und von dieser an den Zugang für die 2D-Übersichtskameras 44 und 50, an den Zugang für die 3D-Detailkamera 46, an den Zugang 48, an die Vorpositioniereinrichtung 45, 47, 49, 51 sowie an die Bogenführung 43 zur weiteren Verarbeitung gesendet werden. Die Steuereinheit 36 ist über eine geeignete Datenverbindung 37 mit dem Trägersystem verbunden. Der OP-Tisch 39 kann steuerungstechnisch über die Datenverbindung 38 ebenfalls mit der Steuereinheit 36 verbunden sein, um bei einer Veränderung der OP-Tisch - Position, z.B. der Höhe, diese Positionsänderung in der Steuereinheit zu verarbeiten und zu signalisieren. Damit können Veränderungen der Patientenposition aufgrund einer Positionsveränderung des OP-Tisches 39 ausgewertet werden.

Die aufgenommen Bilddaten werden über die Datenstrecken 29a, 29b, 30 einer Verarbeitungseinheit 31 zugeführt, welche die Bilddaten zur Darstellung aufbereitet und über eine weitere Datenstrecke 32 einer Visualisierungseinheit 33 zuführt. Die Visualisierungseinheit 33 kann sowohl 2D- als auch 3D-Bilddaten darstellen, beispielsweise getrennt, jedoch auch kombiniert in einem einzigen Bild bzw. einer einzigen Bildabfolge.

Die Bedien- und Anzeigeeinheit 34 ist über eine geeignete Datenverbindung 52 mit der Verarbeitungseinheit 31 gekoppelt. Der Chirurg kann über die Bedien- und Anzeigeeinheit 34 Steuerbefehle zur Auswahl, Verarbeitung und Darstellung der Bilddaten an die Verarbeitungseinheit 31 senden. Die Verarbeitungseinheit 31 ist mittels einer geeigneten Datenverbindung 32 mit der Anzeigeeinheit 33 verbunden. Die Anzeigeeinheit 33 kann die von der 2D-Übersichtskamera und der 3D-Detailkamera gelieferten Bilder / Bildfolgen sowie zusätzliche, in der Verarbeitungseinheit 31 erzeugte Informationen, wie z.B. Trajektorien der chirurgischen Instrumente, entweder als separate Bilder / Bildfolge oder als mit den Bildinformationen der 2D-Übersichtskamera und/oder der 3D-Detailkamera verrechnete Bilder / Bildfolgen darstellen.

So beschreibt die vorliegende Erfindung ein chirurgisches Robotersystem, bei dem die Trajektoren der chirurgischen Instrumente bzw. der Beleuchtungseinrichtungen in der Anzeigeeinheitsbeziehung auf einem Display angezeigt werden, so dass der Chirurg neben der aktuellen Position von einzelnen Elementen der Instrumente auch noch virtuell angezeigt bekommt, in welcher Richtung sich weitere Instrumente bzw. Beleuchtungseinrichtungen befinden. Dadurch möglichst die vorliegende Erfindung, dass der Chirurgen stets alle Instrumente koordinieren kann und nicht im Blindflugflug zu dem Sichtbereich der 3D-Kamera zuführen muss.

## Patentansprüche

1. Chirurgisches Robotersystem mit zumindest zwei Roboterarmen (45, 47, 49, 51) an welchen jeweils zumindest ein Endoskop für die minimalinvasive Chirurgie angeordnet sind,
wobei das erste Endoskop an dem ersten Roboterarm (47) eine erste Hauptträgereinrichtung (4b), die sich im Wesentlichen über die gesamte Endoskoplänge von außen in das Körperinnere erstreckt, und welche am distalen Ende zumindest eine Beleuchtungseinheit (23, 24) und zwei Bildaufnahmeeinrichtungen (20a, 21a, 22a, 20b, 21b, 22b) aufweist, wobei die Bildaufnahmeeinrichtungen (20a, 21a, 22a, 20b, 21b, 22b) jeweils im Wesentlichen in der gleichen Ebene von der ersten Hauptträgereinrichtung (4b) nach außen schwenkbar angeordnet sind, und einen ersten Trokar (1b) umfasst, welcher den Zugang des ersten Endoskops in das Körperinnere bewerkstelligt, und
wobei das zweite Endoskop an dem zweiten Roboterarm (45) eine zweite Hauptträgereinrichtung (4a) für ein chirurgisches Instrument, die sich im Wesentlichen über die gesamte Endoskoplänge von außen in das Körperinnere erstreckt,
einen zweiten Trokar (1a), welcher den Zugang des zweiten Endoskops in das Körperinnere bewerkstelligt, und
eine erste Zusatzträgereinrichtung (3) aufweist, die an der zweiten Hauptträgereinrichtung (4a) vorgesehen ist, wobei die Zusatzträgereinrichtung (3) an ihrem distalen Ende eine Zusatzbildaufnahmeeinrichtung (8, 9, 10, 11) aufweist, die von der Zusatzträgereinrichtung (3) nach außen schwenkbar angeordnet ist und wobei die Zusatzbildaufnahmeeinrichtung (8, 9, 10, 11) eine Zusatzbeleuchtungseinheit (10, 11) und zumindest einen Zusatzbildsensor (8, 9) aufweist, welcher einen Überwachungsbereich aufweist, der die beiden Überwachungsbereiche der Bildaufnahmeeinrichtungen (20a, 21a, 22a, 20b, 21b, 22b) des ersten Endoskops umfasst,
wobei eine Bildverarbeitungseinheit (31), welche sowohl mit den beiden Bildaufnahmeeinrichtungen (20a, 21a, 22a, 20b, 21b, 22b) als auch der Zusatzbildaufnahmeeinrichtung (8, 9, 10, 11) gekoppelt ist, und eine Visualisierungseinheit (33) vorgesehen ist, welche 2D-Bilddaten und/oder 3D-Bilddaten der Bildaufnahmeeinrichtungen (20a, 21a, 22a, 20b, 21b, 22b) und/oder der Zusatzbildaufnahmeeinrichtung (8, 9, 10, 11) darstellt,
wobei die Zusatzträgereinrichtung (3) zwischen dem Trokar (1a) und der zweiten Hauptträgereinrichtung (4a) vorgesehen ist, insbesondere unmittelbar an der zweiten Hauptträgereinrichtung (4a) anliegt, wobei insbesondere sowohl die zweite Hauptträgereinrichtung (4a) als auch die Zusatzträgereinrichtung (3) zylinderförmig ausgebildet sind.

2. Robotersystem nach Anspruch 1, **dadurch gekennzeichnet, dass** der Zusatzbildsensor (8, 9) eine Weitwinkeloptik (8) aufweist, welcher im ausgeschwenkten Zustand nahe an dem distalen Ende des zweiten Trokars (1a) angeordnet ist.

3. Robotersystem nach Anspruch 1 oder 2, **dadurch gekennzeichnet, dass** die beiden Bildaufnahmeeinrichtungen (20a, 21a, 22a, 20b, 21b, 22b) jeweils am distalen Ende der ersten Hauptträgereinrichtung (4b) um eine Schwenkachse (25a, 25b) schwenkbar angeordnet sind, wobei die Schwenkachsen (25a, 25b) parallel zueinander in einer Ebene liegen.

4. Robotersystem nach einem der Ansprüche 1 bis 3, **dadurch gekennzeichnet, dass** die beiden Bildaufnahmeeinrichtungen (20a, 21a, 22a, 20b, 21b, 22b) mittels Gelenken (25a, 25b) jeweils sowohl um eineSchwenkachse (25a, 25b) als auch um eine weitere Drehachse (27, 28) orthogonal zur Längserstreckung der ersten Hauptträgereinrichtung (4b) kippbar angeordnet sind, wobei die Drehbewegungen um die Schwenkachsen (25a, 25b) und die Drehachsen (27, 28) voneinander unabhängig entkoppelt sind.

5. Robotersystem nach einem der Ansprüche 1 bis 4, **dadurch gekennzeichnet, dass** zumindest ein drittes Endoskop an einem dritten Roboterarm (51) vorgesehen ist, welches eine dritte Hauptträgereinrichtung (4d) für ein chirurgisches Instrument, die sich im Wesentlichen über die gesamte Endoskoplänge von außen in das Körperinnere erstreckt,
einen dritten Trokar (1d), welcher den Zugang des dritten Endoskops in das Körperinnere bewerkstelligt, und
eine zweite Zusatzträgereinrichtung (3d) aufweist, die an der dritten Hauptträgereinrichtung (4d) vorgesehen ist, wobei die zweite Zusatzträgereinrichtung (3d) an ihrem distalen Ende eine zweite Zusatzbildaufnahmeeinrichtung (8d, 9d, 10d, 11d) aufweist, die von der zweiten Zusatzträgereinrichtung (3d) nach außen schwenkbar angeordnet ist und wobei die zweite Zusatzbildaufnahmeeinrichtung (8d, 9d, 10d, 11d) eine Zusatzbeleuchtungseinheit (10d, 11d) und zumindest einen zweiten Zusatzbildsensor (8d, 9d) aufweist, welcher einen zweiten Überwachungsbereich aufweist, der die beiden Überwachungsbereiche der beiden Bildaufnahmeeinrichtungen (20a, 21a, 22a, 20b, 21b, 22b) des ersten Endoskops umfasst.

6. Robotersystem nach Anspruch 5, **dadurch gekennzeichnet, dass** die zweite Zusatzbildaufnahmeeinrichtung (8d, 9d, 10d, 11d) des dritten Endoskops mit der Bildverarbeitungseinheit (31) gekoppelt ist, und die Visualisierungseinheit (33) die 2D-Bilddaten und/oder 3D-Bilddaten der beiden Bildaufnahmeeinrichtungen (20a, 21a, 22a, 20b, 21b, 22b) und/oder der ersten Zusatzbildaufnahmeeinrichtung (8, 9, 10, 11) und/oder der zweiten Zusatzbildaufnahmeeinrichtung (8d, 9d, 10d, 11d) darstellt.

## Claims

1. A surgical robot system having at least two robot arms (45, 47, 49, 51) on each of which at least one endoscope for minimally invasive surgery is arranged,
wherein the first endoscope on the first robot arm (47) comprises a first main support means (4b) which essentially extends over the entire endoscope length from outside into the interior of the body and which comprises at the distal end at least one lighting unit (23, 24) and to image-taking devices (20a, 21a, 22a, 20b, 21b, 22b), wherein the image-taking devices (20a, 21a, 22a, 20b, 21b, 22b) are each arranged in essentially the same plane so as to be pivotable outwards from the main support means (4b), and comprises a first trocar (1b) which effects access of the first endoscope into the interior of the body and
wherein the second endoscope on the second robot arm (45) comprises a second main support means (4a) for a surgical instrument which extends essentially over the entire endoscope length from outside into the interior of the body,
a second trocar (1a) which effects access of the second endoscope into the interior of the body and
a first auxiliary support means(3) which is provided on the second main support means (4a), wherein the auxiliary support means (3) comprises at its distal an additional image-taking device(8, 9, 10, 11) which is arranged pivotally outwards from the auxiliary support means (3) and wherein the additional image-taking device (8, 9, 10, 11) has an auxiliary lighting unit (10, 11) and at least one additional image sensor (8, 9) which has a monitoring area which includes the two monitoring areas of the image-taking device (20a, 21a, 22a, 20bm 21b, 22b) of the first endoscope,
wherein an image processing unit (31), which is coupled to both to the two image-taking devices (20a, 21a, 22a, 20b, 22b, 22b) as well as the additional image-taking unit (8, 9, 10, 11) and a visualisation unit (33) is provided, which displays 2D image data and/or 3D image data of the image-taking devices (20a, 21a, 22a, 20b, 21b, 22b) and/or of the additional image-taking device (8, 9, 10, 11),
wherein the auxiliary support means (3) is provided between the trocar (1a) and the second main support means (4a), in particular directly adjoins the second main support means (4a), wherein, in particular, both the second main support means (4a) and the auxiliary support means (3) are cylindrical in design.

2. The robot system according to claim 1, **characterised in that** the additional image sensor (8, 9) has a wide-angle lens (8) which in the pivoted state is arranged close to the distal end of the second trocar (1a).

3. The robot system according to claim 1 or 2, **characterised in that** the two image-taking devices (20a, 21a, 22a, 20b, 21b, 22b) are each arranged pivotally about a pivot axis (25a, 25b) at the distal end of the first support device (4b) wherein the pivot axes (25a, 25b) are in parallel to each other in one plane.

4. The robot system according to any one of claims 1 to 3, **characterised in that** two image-taking devices (20a, 21a, 22a, 20b, 21b, 22b) are each arranged by means of joints (25a, 25b) in a tilting manner about a pivot axis (25a, 25b) as well as about a further axis of rotation (27, 28) orthogonal to the longitudinal extent of the first main support means (4b), wherein the rotational movements about the pivot axes (25a, 25b) and the axes of rotation (27, 28) are independent being decoupled from each other.

5. The robot system according to any one of claims 1 to 4, **characterised in that** at least one third endoscope is provided on a third robot arm (51) which has a third main support means (4d) for a surgical instrument extending essentially over the entire length of the endoscope from outside into the interior of the body,
a third trocar (1d) which effects access of the third endoscope into the interior of the body and
a second auxiliary support means (3d) which is provided on the third main support means (4d), wherein the second auxiliary support means (3d) has at its distal end a second additional image-taking device (8d, 9d, 10d, 11d), which arranged pivotably from the second auxiliary support means (3d) to the outside and wherein the second additional image-taking device (8d, 9d, 10d, 11d) has an additional lighting unit (10d, 11d) and at least one second additional image sensor (8d, 9d) which has a second monitoring area which includes the two monitoring areas of the two image-taking devices (20a, 21a, 22a, 20b, 21b, 22b) of the first endoscope.

6. The robot system according to claim 5, **characterised in that** the second additional image-taking device (8d, 9d, 10d, 11d) of the third endoscope is coupled with the image processing unit (31), and the visualisation unit (33) displays the 2D image data and/or 3D image data of the two image-taking devices (20a, 21a, 22a, 20b, 21b, 22b) and/or the first additional image-taking device (8 ,9, 10, 11) and/or the second image-taking device (8d, 9d, 10d, 11d).

## Revendications

1. Système de chirurgie robotique, comportant au moins deux bras de robot (45, 47, 49, 51) sur chacun desquels est placé au moins un endoscope, destiné à la chirurgie à invasion minimale,
le premier endoscope réalisant sur le premier bras de robot (47) un premier système de support principal (4b), qui s'étend sur sensiblement toute la longueur de l'endoscope, de l'extérieur vers l'intérieur du corps et qui sur l'extrémité distale comporte au moins un module d'éclairage (23, 24) et deux systèmes d'enregistrement d'image (20a, 21a, 22a, 20b, 21b, 22b), les systèmes d'enregistrement d'image (20a, 21a, 22a, 20b, 21b, 22b) étant placés en tant susceptible de pivoter vers l'extérieur chacun sensiblement dans le même plan du système de support principal (4b) et comprenant un premier trocart (1b) qui assure l'accès du premier endoscope à l'intérieur du corps et
le deuxième endoscope réalisant sur le deuxième bras de robot (45) un deuxième système de support principal (4a) pour un instrument chirurgical, qui s'étend sur sensiblement toute la longueur de l'endoscope, de l'extérieur vers l'intérieur du corps,
un deuxième trocart (1a), lequel représente un deuxième endoscope à l'intérieur du corps,
comportant un premier système de support additionnel (3) qui est prévue sur le deuxième système de support principal (4a), sur son extrémité distale, le système de support additionnel (3) comportant un système additionnel d'enregistrement d'image (8, 9, 10, 11) qui est placé en étant susceptible de pivoter vers l'extérieur, à partir du système de support additionnel (3) et le système additionnel d'enregistrement d'image (8, 9, 10, 11) comprenant un module d'éclairage additionnel (10, 11) et au moins un capteur d'image additionnel (8, 9), qui comportent chacun des zones de supervision d'un système d'enregistrement d'image (20a, 21a, 22a, 20b, 21b, 22b) du premier endoscope,
un module de traitement d'images (31), qui est connecté aussi bien sur les deux systèmes d'enregistrement d'image (20a, 21a, 22a, 20b, 21b, 22b) qu'également sur le système additionnel d'enregistrement d'image (8, 9, 10, 11) et un système de visualisation (33) étant prévus, lequel comporte données des données d'image en images en 2D et/ou des données d'image en 3D du systèmes d'enregistrement d'image (20a, 21a, 22a, 20b, 21b, 22b) et/ou représente le système additionnel d'enregistrement d'image (8, 9, 10, 11),
le système de support additionnel (3) étant prévu entre le trocart (1a) et le deuxième système de support principal (4a), notamment directement sur le deuxième système de support principal (4a), notamment aussi bien le deuxième système de support principal (4a) qu'également me système de support additionnel (3) étant conçus sous forme cylindrique.

2. Système robotique selon la revendication 1, **caractérisé en ce que** le capteur d'image additionnel (8, 9) comporte une optique à grand angle (8) laquelle, lorsqu'elle est déployée est placée sur l'extrémité distale du deuxième trocart (1a).

3. Système robotique selon la revendication 1 ou 2, **caractérisé en ce que** les deuxièmes systèmes d'enregistrement d'image (20a, 21a, 22a, 20b, 21b, 22b) sont placés chacun sur l'extrémité distale du premier système de support principal (4b) en étant susceptibles de pivoter autour d'un axe de pivotement (25a, 25b), les axes de pivotement (25a, 25b) se situant à la parallèle l'un de l'autre dans un plan.

4. Système robotique selon l'une quelconque des revendications 1 à 3, **caractérisé en ce que** les deux systèmes d'enregistrement d'image (20a, 21a, 22a, 20b, 21b, 22b) sont placés en étant susceptibles de pivoter au moyen d'articulations 25a, 25b), chacun aussi bien autour d'un axe de pivotement (25a, 25b) qu'aussi bien autour d'un axe de rotation (27, 28) supplémentaire, en direction orthogonale à l'extension longitudinale du premier système de support principal (4b), les déplacements en rotation autour des axes de pivotement (25a, 25b) et des axes de rotation (27, 28) étant désaccouplés l'un de l'autre.

5. Système robotique selon l'une quelconque des revendications 1 à 4, **caractérisé en ce qu'**il est prévu au moins un troisième endoscope sur un troisième bras de robot (51), lequel comporte un troisième système de support principal (4d) pour un instrument chirurgical, qui s'étend sensiblement sur toute la longueur de l'endoscope, de l'extérieur vers l'intérieur du corps,
un troisième trocart (1d), lequel assure l'accès du troisième endoscope vers l'intérieur du corps, et
un deuxième système de support additionnel (3d) qui est prévu sur le troisième système de support principal (4d), sur son extrémité distale, le deuxième système de support additionnel (3d) comportant un deuxième système additionnel d'enregistrement d'image (8d, 9d, 10d, 11d) qui est placé en étant susceptible de pivoter vers l'extérieur, autour du deuxième système de support additionnel (3d) et le deuxième système additionnel d'enregistrement d'image (8d, 9d, 10d, 11d) comprenant un module d'éclairage additionnel (10d, 11d) et au moins un deuxième capteur d'image additionnel (8d, 9d), lequel comporte une deuxième zone de supervision qui comprend les deux zones de supervision des deux systèmes d'enregistrement d'image (20a, 21a, 22a, 20b, 21b, 22b) du premier endoscope.

6. Système robotique selon la revendication 5, **caractérisé en ce que** le deuxième système additionnel d'enregistrement d'image (8d, 9d, 10d, 11d) du troisième endoscope est couplé sur le module de traitement d'images (31), et **en ce que** le module de visualisation (33) représente les données d'image en 2D et/ou les données d'image en 3D des deux systèmes d'enregistrement d'image (20a, 21a, 22a, 20b, 21b, 22b) et/ou du premier système additionnel d'enregistrement d'image (8, 9, 10, 11) et/ou du deuxième système additionnel d'enregistrement d'image (8d, 9d, 10d, 11d).
